(19) Europäisches Patentamt
European Patent Office
Office européen des brevets

(11) EP 0 520 873 B1

(12) **FASCICULE DE BREVET EUROPEEN**

(45) Date de publication et mention de la délivrance du brevet: **20.03.1996 Bulletin 1996/12**

(51) Int. Cl.$^6$: **C07D 233/60**, A01N 43/50, A61K 31/415, C11D 1/58

(21) Numéro de dépôt: **92401747.8**

(22) Date de dépôt: **23.06.1992**

(54) **Composés alkylthiopoly(éthylimidazolium), procédé de préparation et leur utilisation comme agents biocides**

Alkylthiopoly(Ethylimidazolium)verbindungen, Verfahren zur Herstellung und deren Verwendung als Biozidemittel

Alkylthiopoly(ethylimidazolium)compounds, process of preparation and their use as biocide agents

(84) Etats contractants désignés:
**AT BE CH DE DK ES FR GB GR IT LI NL PT SE**

(30) Priorité: **24.06.1991 FR 9107735**

(43) Date de publication de la demande:
**30.12.1992 Bulletin 1992/53**

(73) Titulaire: **L'OREAL**
**F-75008 Paris (FR)**

(72) Inventeurs:
- **Sebag, Henri**
  **F-75016 Paris (FR)**
- **Mahieu, Claude**
  **F-75017 Paris (FR)**

(74) Mandataire: **Casalonga, Axel et al**
**BUREAU D.A. CASALONGA - JOSSE**
**Morassistrasse 8**
**D-80469 München (DE)**

(56) Documents cités:
**EP-A- 0 000 752**
**EP-A- 0 331 230**
**WO-A-90/04918**
**FR-A- 2 010 024**
**FR-A- 2 324 721**
**US-A- 3 882 136**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 520 873 B1

Printed by Rank Xerox (UK) Business Services
2.9.13/3.4

## Description

La présente invention concerne de nouveaux composés du type alkylthiopoly(éthylimidazolium), leur procédé de préparation et leur utilisation comme agents biocides dans divers domaines techniques comme la cosmétique, la pharmacie humaine et vétérinaire, l'agriculture, les peintures et vernis, la papeterie.

On recherche en cosmétique des produits ayant des propriétés bactéricides et/ou fongicides et une bonne tolérance vis-à-vis de la peau et des cheveux, notamment dans les produits anti-pelliculaires, dans les produits de nettoyage de la peau.

En dermopharmacie, l'utilisation des produits bactéricides et/ou fongicides présente également un grand intérêt, notamment dans le traitement des maladies affectant la couche cornée de l'épiderme de l'homme ou de l'animal, tel que l'acné ou dans le traitement des mycoses ou encore dans le traitement des maladies affectant les muqueuses.

Les composés cationiques du type ammonium quaternaire sont couramment utilisés comme agents bactéricides en cosmétique ou pharmacie. Ces composés présentent cependant des problèmes de tolérance.

On connaît, dans l'état de la technique, le bromure de cétyltriméthylammonium plus connu sous le nom de "CETAVLON".

La demanderesse a découvert de nouveaux composés dérivés de l'imidazole, présentant une bonne activité biocide, ainsi qu'une toxicité amoindrie par rapport aux composés connus. Ils présentent de plus, de bonnes propriétés cosmétiques vis-à-vis des cheveux, de la peau et des ongles.

Ces composés possèdent par ailleurs des propriétés tensio-actives intéressantes.

La présente invention a pour objet de nouveaux composés cationiques du type imidazolium.

Un autre objet de l'invention consiste en un procédé de préparation de ces composés.

L'invention concerne également l'utilisation de ces composés comme agents biocides dans de nombreux domaines de l'industrie chimique, et plus particulièrement la cosmétique et la dermopharmacie.

D'autres objets apparaîtront à la lumière de la description et des exemples qui suivent.

Les composés conformes à la présente invention répondent à la formule suivante (I) :

$$R_1 - \underset{\underset{(O)_w}{\|}}{S} - (C_5H_6N_2R^+ X^- )_n - H \qquad (I)$$

dans laquelle

- $R_1$ désigne un radical alkyle linéaire ou ramifié, ayant 12 à 18 atomes de carbone;
- R désigne un radical méthyle, éthyle, hydroxyéthyle, benzyle;
- $X^-$ désigne un anion minéral ou organique;
- n est un nombre entier ou décimal compris entre 2 et 15;
- w vaut 0, 1 ou 2;

le groupement $[C_5H_6N_2R^+]$ représentant les structures suivantes prises en mélange ou séparément :

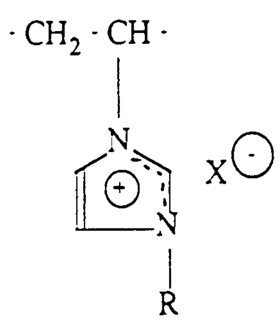
;
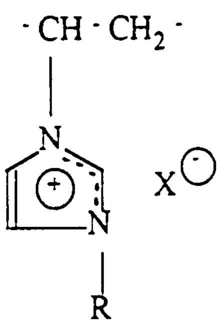

Les anions $X^-$ sont choisis notamment parmi les halogénures, les alkylsulfates, les alkylsulfonates, les arylsulfonates.

L'anion $X^-$ désigne de préférence $Cl^-$, $Br^-$, $I^-$, $CH_3SO_3^-$, $CH_3OSO_3^-$, $C_2H_5OSO_3^-$ ou

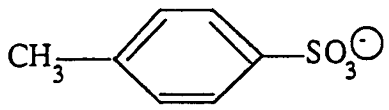

Les composéspréférentiels selon la présente invention sont choisis parmi ceux de formule (I), dans laquelle R désigne un radical méthyle et $X^-$ l'anion $CH_3OSO_3^-$, et R désigne un radical benzyle et $X^-$ l'anion $Cl^{\ominus}$.

Les composés selon l'invention peuvent être préparés par addition radicalaire d'un groupe alkylthio sur une ou plusieurs molécules de 1-vinylimidazole, pour obtenir un tensio-actif alkylthiopoly(éthylimidazole) de formule (II) :

$$R_1S(C_5H_6N_2)_n - H \qquad (II)$$

dans laquelle $R_1$ a la même signification indiquée ci-dessus, le groupement $C_5H_6N_2$ signifiant les structures suivantes prises en mélange ou séparément :

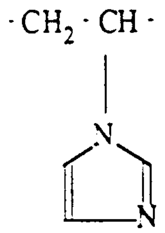 ; 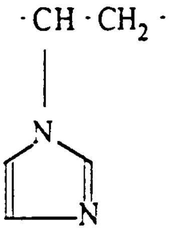

Le composé alkylthiopoly(éthylimidazole) ainsi obtenu est ensuite quaternisé par alcoylation avec un composé de formule RX, dans laquelle R et X ont les significations indiquées ci-dessus.

Dans le cas où w est égal à 1 ou 2, les produits ainsi obtenus sont oxydés à l'eau oxygénée, selon un procédé connu, à une température comprise entre 20° et 50°C.

Le procédé de préparation des composés de l'invention peut être représenté par le schéma réactionnel suivant :

## SCHEMA A

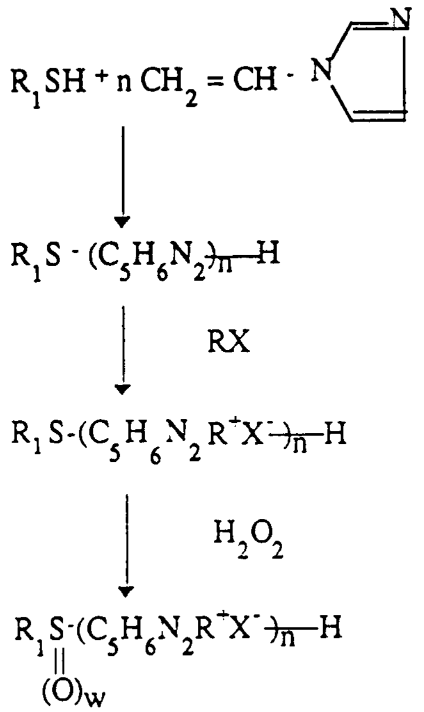


(II)

(I)

La réaction radicalaire a lieu en milieu solvant en présence d'un initiateur de radicaux libres.

Comme initiateurs de radicaux libres, on peut citer les hydroperoxydes tels que l'hydroperoxyde de tertiobutyle, les peroxydes tels que le peroxyde de dibenzoyle, les peresters tels que le peroxybenzoate de tertiobutyle ou les dérivés azoïques et en particulier l'azobisisobutyronitrile.

Les solvants utilisables doivent être inertes vis-à-vis des réactifs et peuvent être choisis parmi les alcools en $C_1$-$C_4$ tels que le méthanol, l'isopropanol, les alkyléthers, les éthers de glycol, les éthers cycliques tels que le tétrahydrofurane, les hydrocarbures aliphatiques ou aromatiques en $C_6$-$C_8$ comme le toluène.

Le mercaptan de formule $R_1SH$ est dissous dans le solvant en présence du 1-vinylimidazole, puis l'initiateur de radicaux est additionné, la réaction étant effectuée sous atmosphère inerte. Les composés de formule (II) ainsi obtenus sont ensuite alcoylés avec un alcoylant RX en présence d'un solvant inerte tel que ceux cités précédemment.

Les alcoylants RX utilisés conformément à l'invention sont choisis par exemple parmi les halogénures de méthyle, d'éthyle ou d'hydroxyéthyle, les sulfates de méthyle ou d'éthyle, le sulfonate de méthyle ou le paratoluènesulfonate de méthyle.

Les composés de formule (II) sont nouveaux et constituent un autre objet de l'invention.

Les composés alkylthiopoly(éthylimidazolium) de formule (I) de l'invention possèdent de bonnes propriétés biocides.

Une bonne activité biocide a été observée selon les méthodes classiques sur les souches suivantes : Pseudomonas aeruginosa, Staphylococcus aureus, Candida albicans. Ces souches peuvent être considérées comme représentatives des principales bactéries et fongi responsables des affections cutanées d'origine bactérienne, mycobactérienne ou dues à l'implantation de levures pathogènes.

La faible toxicité des composés de l'invention a été observée par hémolyse des hématies d'échantillons de sang.

Les composés conformes à la présente invention peuvent être utilisés comme agents biocides ou comme agents conservateurs dans le domaine de l'industrie chimique, notamment dans les produits cosmétiques et l'industrie pharmaceutique à usage humain ou vétérinaire, les produits agricoles, les peintures et les vernis, la papeterie.

Les composés de formule (I) tels que définis précédemment, ont la particularité de se fixer sur les matières kératiniques telles que la peau, les cheveux et les ongles.

Les composés de l'invention peuvent être utilisés comme tensio-actifs.

Leur caractère amphiphile cationique leur confère en plus des propriétés de conditionnement telles que de démêlage, de douceur, de brillance et de souplesse vis-à-vis des cheveux et des propriétés de douceur vis-à-vis de la peau.

Les composés de l'invention sont particulièrement intéressants pour les soins cosmétiques des matières kératiniques. Ils sont également particulièrement intéressants pour le traitement des affections cutanées d'origine bactérienne, mycobactérienne ou dues à l'implantation de levures pathogènes. Ils peuvent notamment être utilisés dans les compositions pharmaceutiques pouvant être appliquées par voie topique sur les muqueuses ou sur la peau pour le traitement de l'acné ou des mycoses ou dans des compositions cosmétiques, notamment des déodorants corporels ou des bains de bouche.

Ils sont également utilisés dans les compositions capillaires pour l'élimination des pellicules.

Un autre objet de l'invention consiste donc en des compositions pharmaceutiques ou cosmétiques pour le traitement ou le soin des matières kératiniques humaines, contenant dans un milieu physiologiquement acceptable, une quantité efficace de composés de formule (I) telle que définie précédemment.

Les composés de formule (I) sont présents dans des concentrations comprises de préférence entre 0,1 et 10% en poids par rapport au poids total de la composition et de préférence entre 0,2 et 5% en poids.

Ces compositions peuvent être des solutions aqueuses, alcooliques ou hydro-alcooliques, des émulsions se présentant sous forme de lait ou de crème, de mousse, de gel, de pâte, de stick ou encore sous forme de spray.

Ces compositions peuvent être pressurisées dans des dispositifs aérosols, en présence d'un agent propulseur, éventuellement en présence de générateurs de mousse ou d'agents émulsionnants.

Comme agents propulseurs, on peut citer les agents de type fréons, les alcanes en $C_3$-$C_5$, des solvants chlorés tels que le chlorure de méthylène ou les éthers comme le diméthyléther.

Les compositions peuvent également se présenter sous forme de dispersion vésiculaire à base de lipides ioniques (liposomes) ou nonioniques.

Ces compositions peuvent contenir de l'eau, un solvant physiologiquement acceptable ou un mélange d'eau et de ce solvant, le solvant étant choisi parmi les alcools inférieurs en $C_1$-$C_4$ tels que l'éthanol, l'isopropanol, le propanol ou les polyalcools tels que le propylèneglycol ou la glycérine; ces solvants étant présents dans des proportions comprises entre 0 et 50%.

Les compositions selon l'invention peuvent contenir également des huiles, des cires naturelles ou synthétiques, des produits tensio-actifs moussants, émulsionnants, dispersants, non-ioniques, cationiques, faiblement anioniques, amphotères ou zwittérioniques, des alcools gras, des silicones, des polymères d'origine naturelle comme les dérivés de cellulose, de guar, de chitosane, des peptides, des polymères de synthèse, des conditionneurs, des stabilisants de mousse, des épaississants, des nacrants, des stérols, des sels, des filtres solaires, des parfums, des colorants, des hydratants, des conservateurs autres que ceux de formule (I), notamment ceux de la famille des isothiazolones tels que la 2-méthylisothiazolone, 2-octylisothiazolone, 5-chloro 2-méthylisothiazolone, benzo-isothiazolone, ou celles décrites dans le brevet FR-2.492.376.

Une forme d'utilisation cosmétique préférentielle est constituée par des shampooings ou des lotions anti-pelliculaires.

Un autre objet de l'invention consiste en un procédé de traitement cosmétique des cheveux ou du cuir chevelu, caractérisé par le fait qu'on applique une composition capillaire telle que définie précédemment et éventuellement à faire suivre d'un rinçage à l'eau.

On observe que les cheveux ainsi traités sont plus souples, plus brillants, plus lisses et facilement démêlables.

Les applications plus particulièrement visées par l'invention sont les shampooings, les traitements des cheveux et du cuir chevelu pour l'élimination des pellicules, le traitement de la peau contre l'acné et les mycoses.

La présente invention a également pour objet l'utilisation des composés de formule (I) pour la préparation d'un médicament destiné au traitement des maladies d'origine bactérienne, mycobactérienne ou dues à des implantations de levures pathogènes.

Les exemples qui suivent servent à illustrer la présente invention sans toutefois présenter un caractère limitatif.

EXEMPLES DE PREPARATION

EXEMPLE 1

Préparation d'un composé de formule (I) pour lequel
$R_1=C_{12}H_{25}$ $\bar{n}=2$ $R=CH_3$ $X=CH_3OSO_3^-$ , w = O

ETAPE 1

Préparation d'un composé de formule (II) pour lequel
$R_1=C_{12}H_{25}$ $\bar{n}$ = 2

Dans un réacteur de 500 ml, on dissout à 25°C, 20,24 g (0,1 mole) de dodécanethiol dans 20 ml d'isopropanol.

La solution est placée sous courant d'azote puis 28,2 g de 1-vinylimidazole (0,3 mole) sont ajoutés en 5 minutes.

Le chauffage est alors amorcé. Lorsque la température atteint 50°C, on additionne goutte à goutte une solution de 1,13 g d'azobisisobutyronitrile dans 40 ml d'isopropanol en 45 minutes, tout en poursuivant la montée progressive en température.

Lorsque l'addition est terminée, le milieu réactionnel est maintenu à une température de 70°C pendant 16 heures.

Le solvant est alors évaporé sous pression réduite. 48,5 g d'un produit brut sont alors obtenus.

Le terme $\bar{n}$=2 est alors séparé de ce produit brut par passage sur silice (Merck 60 H - éluant $CH_2Cl_2$ progressivement enrichi en méthanol pour atteindre un mélange $CH_2Cl_2/CH_3OH$ - 95/5).

6 g du produit de dicondensation sont ainsi obtenus. Le produit se présente sous la forme d'une pâte beige.

Indice de basicité = 5,10 meq/g (Théorique : 5,12 meq/g).

| ANALYSE ELEMENTAIRE | | |
|---|---|---|
| | THEORIQUE | TROUVE |
| C | 67,64 | 67,61 |
| H | 9,81 | 9,85 |
| N | 14,34 | 14,40 |
| S | 8,21 | 8,20 |

ETAPE 2

Quaternisation du composé obtenu à l'étape 1

5,31 g du composé de l'étape 1 sont solubilisés à 25°C dans 2,5 ml de méthanol.

Lorsque la solution est homogène, on ajoute goutte à goutte, en 45 minutes, 3,41 g de sulfate de diméthyle en évitant que la température ne dépasse 50°C.

Le mélange est alors agité 14 h à 25°C.

Le solvant est alors évaporé sous pression réduite. On obtient un produit amorphe de couleur orangée qui est soluble dans l'eau.

| ANALYSE ELEMENTAIRE | | |
|---|---|---|
| | THEORIQUE | TROUVE |
| C | 48,57 | 48,04 |
| H | 7,84 | 7,89 |
| N | 8,72 | 8,58 |
| O | 19,91 | 21,21 |
| S | 14,96 | 14,77 |

EXEMPLE 2

Préparation d'un composé de formule (I) pour lequel
$R_1=C_{12}H_{25}$ $\bar{n}$=3 R = $CH_3$ X = $CH_3OSO_3^-$ , w = O

ETAPE 1

Préparation d'un composé de formule (II) pour lequel
$R_1=C_{12}H_{25}$ $\bar{n}$=3

Ce composé est préparé selon le mode opératoire de l'exemple 1 (1ère étape).

Le produit brut obtenu est un mélange de composés pour lequel n représente une valeur statistique moyenne de 3. Indice de basicité = 5,74 meq/g (Théorique : 6,21 meq/g)

ETAPE 2

Quaternisation du composé obtenu à la première étape

Le mode opératoire est analogue à celui de l'exemple 1 (étape 2) utilisant :

- 10 g du composé obtenu à la 1ère étape de l'exemple 3 solubilisés dans 30 ml de méthanol
- 7,24 g de sulfate de diméthyle

On obtient 17,2 g d'un produit orangé amorphe soluble dans l'eau.

| ANALYSE ELEMENTAIRE | | |
|---|---|---|
| | THEORIQUE | TROUVE |
| C | 45,95 | 44,50 |
| H | 7,24 | 7,32 |
| N | 9,74 | 9,31 |
| O | 22,24 | 24,15 |
| S | 14,86 | 14,60 |

EXEMPLE 3

Préparation d'un composé de formule (I) pour lequel
$R_1=C_{18}H_{37}$ $\bar{n}=3$ $R=CH_3$ $X=CH_3OSO_3^-$ , w = O

ETAPE 1

Préparation d'un composé de formule (II) pour lequel
$R_1=C_{18}H_{37}$ $\bar{n}=3$
Le mode opératoire est analogue à celui de l'exemple 2 (Etape 1) en utilisant :

- 28,66 g d'octadécanethiol (0,1 mole) dissous dans 50 ml d'isopropanol
- 28,23 g (0,3 mole) de 1-vinylimidazole
- 1,13 g d'azobisisobutyronitrile dans 40 ml d'isopropanol.

On obtient 56,9 g d'une pâte beige dont l'indice de basicité est de 4,20 meq/g.

ETAPE 2

Quaternisation du composé obtenu à la première étape

Le mode opératoire est analogue à celui de l'exemple 1 (Etape 2) en utilisant :

- 20 g du composé de l'étape 1 dissous dans 100 ml de méthanol
- 10,58 g de sulfate de diméthyle.

On obtient 30,60 g d'un solide orangé dont le point de fusion est de 230°C avec décomposition.

| ANALYSE ELEMENTAIRE | | |
|---|---|---|
| | THEORIQUE | TROUVE |
| C | 49,45 | 48,64 |
| H | 7,87 | 7,97 |
| N | 8,87 | 8,60 |
| O | 20,27 | 21,67 |
| S | 13,54 | 13,30 |

EXEMPLE 4

Préparation d'un composé de formule (I) pour lequel $R_1 = C_{12}H_{25}$ $\overline{n} = 5$ $R = CH_3$ $X = CH_3OSO_3^-$ , w = O

ETAPE 1

Préparation d'un composé de formule (II) pour lequel $R_1 = C_{12}H_{25}$ et $\overline{n} = 5$ (dodécanethiol thioéthérifié par cinq motifs 1-éthylimidazole) (statistique)

Le mode opératoire est analogue à celui de l'exemple 2 (Etape 1) en utilisant :

- 20,25 g de dodécanethiol (0,1 mole) dissous dans 20 ml d'isopropanol
- 47 g de 1-vinylimidazole (0,5 mole)
- 1,88 g d'azobisisobutyronitrile dans 100 ml d'isopropanol.

On obtient 65 g d'une pâte beige dont l'indice de basicité est 6,21 meq/g (théorique : 7,44 meq/g).

ETAPE 2

Quaternisation du composé de la première étape

Dodécanethiol thioéthérifié par cinq motifs méthylsulfate de 1-éthyl 3-méthylimidazolium (statistique)

Le mode opératoire est identique à celui de l'exemple 1 (Etape 2) en utilisant :

- 20 g du composé de l'étape 1 dissous dans 100 ml de méthanol
- 15,62 g de sulfate de diméthyle.

On obtient 34 g d'un solide orangé.
$T_f$ = 230°C (décomposition).

EXEMPLE 5

Préparation d'un composé de formule (I) pour lequel $R_1 = C_{12}H_{25}$ $\overline{n} = 10$ $R = CH_3$ $X^- = CH_3OSO_3^-$ , w = O

ETAPE 1

Préparation d'un composé de formule (II) pour lequel $R_1 = C_{12}H_{25}$ $\overline{n} = 10$ (dodécanethiol thioéthérifié par dix motifs 1-éthylimidazole) (statistique)

Le mode opératoire est analogue à celui de l'exemple 2 (Etape 1) en utilisant :

- 10,2 g de dodécanethiol (0,05 mole) dissous dans 10 ml d'isopropanol

- 47 g de 1-vinylimidazole (0,5 mole)
- 1,88 g d'azobisisobutyronitrile dans 100 ml d'isopropanol.

Le produit brut obtenu se présente sous la forme d'une pâte beige dont l'indice de basicité est 6,31 meq/g (théorique : 8,75 meq/g).

ETAPE 2

Quaternisation du composé issu de la première étape

Dodécanethiol thioéthérifié par dix motifs méthylsulfate de 1-éthyl 3-méthylimidazolium (statistique)

Le mode opératoire est identique à celui de l'exemple 1 (Etape 2) en utilisant :

- 20,6 g du composé de l'étape 1 dissous dans 100 ml de méthanol
- 16,38 g de sulfate de diméthyle.

On obtient 35 g d'un solide orangé.
$T_f$ = 240°C (décomposition).

| ANALYSE ELEMENTAIRE | | |
|---|---|---|
| | THEORIQUE | TROUVE |
| C | 40,95 | 39,09 |
| H | 6,12 | 6,79 |
| N | 11,65 | 10,47 |
| O | 26,61 | 28,59 |
| S | 14,67 | 13,81 |

EXEMPLE 6

Préparation d'un composé de formule (I) pour lequel
$R_1 = C_{18}H_{37}$ $\bar{n} = 8$ $R = CH_3$ $X^- = CH_3OSO_3^-$ , w = O

ETAPE 1

Préparation d'un composé de formule (II) pour lequel $R_1 = C_{18}H_{37}$ et $\bar{n} = 8$ (octadécanethiol thioéthérifié par huit motifs 1-éthylimidazole) (statistique)

Dans un réacteur de 500 ml, on dissout, à 70°C et sous courant d'azote, 14,34 g d'octadécanethiol dans 100 ml d'isopropanol. On amène la température à 40°C et on additionne 37,6 g de 1-vinylimidazole (0,4 mole) goutte-à-goutte en 10 minutes. Lorsque l'addition est terminée, le chauffage est repris. Quand la température dans le réacteur atteint 50°C, on additionne une solution de 1,50 g d'azobisisobutyronitrile dans 50 ml de méthanol en 45 minutes tout en poursuivant la montée progressive en température. Lorsque l'addition est terminée, le milieu réactionnel est maintenu à une température de 70°C pendant 16 heures. Les solvants sont ensuite chassés sous pression réduite et on obtient 52 g d'un solide jaune pâle.

Indice de basicité : 6,82 meq/g (théorique : 7,70 meq/g)

| ANALYSE ELEMENTAIRE | | |
|---|---|---|
| | THEORIQUE | TROUVE |
| C | 67,02 | 66,10 |
| H | 8,34 | 8,77 |
| N | 21,56 | 20,26 |
| S | 3,08 | 3,15 |

ETAPE 2

Quaternisation du composé issu de la première étape

Octadécanethiol thioéthérifié par huit motifs méthylsulfate de 1-éthyl 3-méthylimidazolium (statistique)

Le mode opératoire est identique à celui de l'exemple 1 (Etape 2) en utilisant :

- 20 g du composé de l'étape 1 dissous dans 100 ml de méthanol
- 17,19 g de sulfate de diméthyle.

On obtient 36,5 g d'un solide orangé.

| ANALYSE ELEMENTAIRE | | |
|---|---|---|
| | THEORIQUE | TROUVE |
| C | 43,39 | 42,95 |
| H | 6,59 | 6,74 |
| N | 10,94 | 10,70 |
| O | 25,00 | 26,06 |
| S | 14,09 | 13,89 |

EXEMPLE 7

Préparation d'un composé de formule (I) pour lequel
$R_1 = C_{18}H_{37}$ $\bar{n} = 10$ $R = CH_3$ $X^- = CH_3OSO_3^-$ , w = O

ETAPE 1

Préparation d'un composé de formule (II) pour lequel $R_1 = C_{18}H_{37}$ $\bar{n} = 10$ (octadécanethiol thioéthérifié par dix motifs 1-éthylimidazole) (statistique)

Le mode opératoire est analogue à celui de l'exemple 6 (Etape 1) en utilisant :

- 14,37 g d'octadécanethiol (0,05 mole) dissous dans 100 ml d'isopropanol
- 47 g de 1-vinylimidazole (0,5 mole)
- 1,88 g d'azobisisobutyronitrile dans 50 ml de méthanol.

On obtient 61 g d'un solide jaune pâle dont la basicité est 6,98 meq/g (théorique : 8,15 meq/g).

| ANALYSE ELEMENTAIRE | | |
|---|---|---|
| | THEORIQUE | TROUVE |
| C | 66,52 | 66,29 |
| H | 8,05 | 8,93 |
| N | 22,82 | 19,66 |
| S | 2,61 | 2,62 |

ETAPE 2

Quaternisation du composé issu de la première étape

Octadécanethiol thioéthérifié par dix motifs méthylsulfate de 1-éthyl 3-méthylimidazolium (statistique)

Le mode opératoire est identique à celui de l'exemple 1 (Etape 2) en utilisant :

- 20 g du composé issu de l'étape 1 dissous dans 100 ml de méthanol
- 17,39 g de sulfate de diméthyle.

On obtient 36,5 g d'un solide orangé.

| | THEORIQUE | TROUVE |
|---|---|---|
| C | 42,46 | 42,60 |
| H | 6,40 | 4,46 |
| N | 11,26 | 11,13 |
| O | 25,71 | 27,02 |
| S | 14,17 | 13,15 |

EXEMPLE 8

Préparation d'un composé de formule (I) pour lequel
$R_1 = C_{18}H_{37}$ $\overline{n} = 12$ $R = CH_3$ $X^- = CH_3OSO_3^-$ , w = O

ETAPE 1

Préparation d'un composé de formule (II) pour lequel $R_1 = C_{18}H_{37}$ et $\overline{n} = 12$ (octadécanethiol thioéthérifié par douze motifs 1-éthylimidazole) (statistique)

Le mode opératoire est analogue à celui de l'exemple 6(Etape 1) en utilisant :

- 14,37 g d'octadécanethiol (0,05 mole) dissous dans 100 ml d'isopropanol
- 56,4 g de 1-vinylimidazole (0,6 mole)
- 2,16 g d'azobisisobutyronitrile dans 60 ml de méthanol.

On obtient 70 g d'un solide jaune dont la basicité est de 7,35 meq/g (théorique : 8,48 meq/g).

| ANALYSE ELEMENTAIRE | | |
|---|---|---|
| | THEORIQUE | TROUVE |
| C | 66,16 | 66,45 |
| H | 7,83 | 9,08 |
| N | 23,74 | 19,92 |
| S | 2,26 | 2,29 |

ETAPE 2

Quaternisation du composé issu de la première étape

Octadécanethiol thioéthérifié par douze motifs méthylsulfate de 1-éthyl 3-méthylimidazolium (statistique)

Le mode opératoire est identique à celui de l'exemple 1 (Etape 2) en utilisant :

- 20 g du composé issu de l'étape 1 dissous dans 100 ml de méthanol
- 18,2 g de sulfate de diméthyle.

On obtient 36,1 g d'un solide orangé.

| ANALYSE ELEMENTAIRE | | |
|---|---|---|
| | THEORIQUE | TROUVE |
| C | 41,82 | 42,42 |
| H | 6,26 | 6,37 |
| N | 11,48 | 11,62 |
| O | 26,21 | 27,08 |
| S | 14,23 | 13,08 |

EXEMPLE 9

Préparation d'un composé de formule (I) pour lequel

$R_1 = C_{12}H_{25}$ $\bar{n} = 10$ R = benzyle $X^- = Cl^-$ w = O

On solubilise 29 g de composé obtenu à l'étape 1 de l'exemple 5 dans 100 ml de diméthylacétamide à 80°C. On ajoute, à 80°C, 63,85 g de chlorure de benzyle (0,50 mole) en 15 minutes.

Le mélange est alors chauffé 24 heures à 80°C puis ramené à 25°C. Le produit est précipité sous agitation dans 850 ml de tétrahydrofuranne.

Le précipité est filtré, rincé par deux fois 300 ml de tétrahydrofuranne et séché. Le précipité, qui est hygroscopique, est repris par 600 ml d'eau, les dernières traces de tétrahydrofuranne éliminées à l'évaporateur rotatif.

La solution, glacée dans un bain d'éthanol et de carboglace, est ensuite lyophilisée. On obtient 29 g d'une poudre beige ayant une teneur en eau d'environ 9%.

| ANALYSE ELEMENTAIRE | | |
|---|---|---|
| | CALCULE avec 9% d'eau | TROUVE |
| C | 60,03 | 59,33 |
| H | 6,93 | 7,07 |
| N | 10,61 | 10,16 |
| O | 7,78 | 7,21 |
| S | 1,11 | 0,96 |
| Cl | 13,63 | 14,17 |

<u>EXEMPLES DE COMPOSITION</u>

<u>EXEMPLE 1</u>

| CREME EMULSION HUILE/EAU | |
|---|---|
| - Alcool cétylique | 5,0 g |
| - Distéarate de glycéryle | 1,5 g |
| - Monostéarate de glycéryle | 1,5 g |
| - Stéarate de polyéthylèneglycol à 50 moles d'oxyde d'éthylène, vendu sous la dénomination MYRJ 53 par la Société ICI | 3,0 g |
| - Huile de jojoba | 3,0 g |
| - Huile de vaseline | 3,0 g |
| - Paraméthoxycinnamate de 2-éthylhexyle, vendu sous la dénomination PARSOL MCX par la Société GIVAUDAN | 1,0 g |
| - 2-hydroxy 4-méthoxybenzophénone vendu sous la dénomination UVINUL M40 par la Société BASF | 1,0 g |
| - Parfums, conservateur qs | |
| - Composé de l'exemple 2 | 1,0 g |
| - Eau déminéralisée qsp | 100,0 g |

On chauffe les corps gras et les émulsionnants vers 80-85°C. Par ailleurs, on chauffe à 80-85°C l'eau contenant le composé de l'exemple 3 et on ajoute la phase grasse à la phase aqueuse. Après 10 minutes d'agitation vive, on laisse refroidir sous agitation modérée, puis on ajoute le parfum et les conservateurs.

EXEMPLE 2

| CREME EMULSION HUILE/EAU | |
|---|---|
| - Triéthanolamine | 1,3 g |
| - Huile de palme | 3,0 g |
| - Mélange d'éthyl-2 hexanoate de cétostéaryle et de myristate d'isopropyle (90/10) | 3,0 g |
| - Isoparaffine hydrogénée | 10,0 g |
| - Acide stéarique | 2,0 g |
| - Alcool cétylstéarylique | 1,5 g |
| - Myristate d'isopropyle | 3,0 g |
| - Acide polyacrylique réticulé, vendu sous la dénomination CARBOPOL 940 par la Société GOODRICH | 0,6 g |
| - Monostéarate de polyéthylèneglycol (à 50 moles d'oxyde d'éthylène), vendu sous la dénomination MYRJ 53 par la Société ICI | 3,0 g |
| - Mélange de stéarate de glycéryle et de stéarate de polyéthylèneglycol (à 10 moles d'oxyde d'éthylène) (50-50), vendu sous la dénomination ARLACEL 165 par la Société ICI | 3,0 g |
| - Mélange d'alcool cétylstéarylique et d'alcool cétylstéarylique oxyéthyléné à 33 moles d'oxyde d'éthylène, vendu sous la dénomination SINNOWAX AO par la Société HENKEL | 3,0 g |
| - Paraméthoxycinnamate de 2-éthylhexyle, vendu sous la dénomination PARSOL MCX par la Société GIVAUDAN | 1,0 g |
| - 2-hydroxy 4-méthoxybenzophénone, vendu sous la dénomination UVINUL M 40 par la Société BASF | 1,0 g |
| - Parfums, conservateurs qs | |
| - Composé de l'exemple 2 | 1,0 g |
| - Eau déminéralisée qsp | 100,0 g |

Cette émulsion est préparée comme dans l'exemple 2.

EXEMPLE 3

| SHAMPOOING | |
|---|---|
| - Tensio-actif faiblement anionique de formule : $C_{12}H_{25}(O\ CH_2\ CH_2)_n\ O\ CH_2\ COOH$ dans laquelle n = 4 en solution à 95%, vendu sous la dénomination de AKYPO RLM 45 par la Société CHEMY et préparé à 20% de MA neutralisé par la soude à pH 7 | 10,0gMA |
| - Cocoamidoéthyl(N-hydroxyéthyl, N-carboxyméthyl)glycinate de sodium, vendu sous la dénomination MIRANOL C2M concentré NP par la Société MIRANOL | 1,3 g |
| - Composé de l'exemple 5 | 0,1 g |
| - Eau qsp | 100,0 g |
| - pH spontané = 7,5 | |

EXEMPLE 4

| SHAMPOOING | |
|---|---|
| - Cocoamidoéthyl(N-hydroxyéthyl,N-carboxyméthyl)glycinate de sodium, vendu sous la dénomination MIRANOL C2M concentré NP par la Société MIRANOL | 20,0 g |
| - Composé de l'exemple 7 | 0,1 g |
| - Eau qsp | 100,0 g |
| - pH spontané = 8 | |

EXEMPLE 5

| CREME EMULSION HUILE/EAU | |
|---|---|
| - Alcool cétylique | 5,0 g |
| - Distéarate de glycéryle | 1,5 g |
| - Monostéarate de glycéryle | 1,5 g |
| - Stéarate de polyéthylèneglycol à 50 moles d'oxyde d'éthylène, vendu sous la dénomination MYRJ 53 par la Société ICI | 3,0 g |
| - Huile de jojoba | 3,0 g |
| - Huile de vaseline | 3,0 g |
| - Paraméthoxycinnamate de 2-éthylhexyle, vendu sous la dénomination PARSOL MCX par la Société GIVAUDAN | 1,0 g |
| - 2-hydroxy 4-méthoxybenzophénone, vendu sous la dénomination UVINUL M40 par la Société BASF | 1,0 g |
| - Parfums, conservateurs qs | |
| - Composé de l'exemple 9 | 1,0 g |
| - Eau déminéralisée qsp | 100,0 g |

On chauffe les corps gras et les émulsionnants vers 80-85°C. Par ailleurs, on chauffe à 80-85°C l'eau contenant le composé de l'exemple 9 et on ajoute la phase grasse à la phase aqueuse. Après 10 minutes d'agitation vive, on laisse refroidir sous agitation modérée, puis on ajoute le parfum et les conservateurs.

EXEMPLE 6

CREME DE SOIN POUR VISAGE A PEAU GRASSE

On réalise par fusion, à la température de 110°C, le mélange des produits suivants :

- Lipide non-ionique de formule :

$$C_{16}H_{33}O\left[C_3H_5(OH)O\right]_{\overline{n}}H$$

formule où $\overline{n}$ est une valeur statistique moyenne égale à 3 et où $-C_3H_5(OH)O-$ est représenté par les structures suivantes prises en mélange ou séparément : 3,8 g

$-CH_2CHOHCH_2O-$ ,

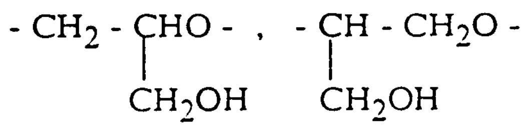

- Cholestérol 3,8 g
- Stéaroylglutamate mono-sodique vendue sous la dénomination ACYLGLUTAMATE HS 11 par la Société AJINOMOTO 0,4 g

Puis on ramène la température du mélange fondu à 90°C. On ajoute progressivement un mélange constitué de 60,6 g d'eau, 3 g de glycérol et 1 g du composé de l'exemple 3 à température de 70°C. On homogénéise le mélange à l'aide d'un ultra-disperseur VIRTIS pendant 6 minutes à la vitesse de 40.000 t/minute. On obtient ainsi une dispersion de vésicules lipides dont la taille moyenne est de 230 nm. On ajoute alors 1 g d'eau contenant 0,15 g de diazolidinylurée vendu sous la dénomination GERMAL II par la Société SUTTON et 0,05 g de sel dipotassique de l'acide éthylène diamino tétracétique.

On ajoute alors :

- 10 g de décaméthyl cyclo pentasiloxane vendue sous la dénomination SILBIONE Huile 700 45 V5 par la Société RHONE POULENC.
- 0,8 g de diméthicone vendue sous la dénomination SILBIONE 747 V 300 par la Société RHONE POULENC
- 0,01 g de paraoxybenzoate de propyle.

On soumet le tout à l'action du disperseur VIRTIS pendant 4 minutes à 40.000 t/minute.

On ajoute :

- 0,42 g d'acide polyacrylique réticulé vendu sous la dénomination CARBOPOL 940 par la Société GOODRICH
- 0,2 g de paraoxybenzoate de méthyle
- 14 g d'eau
- 0,7 g de triéthanolamine.

On obtient ainsi une crème blanche épaisse.

## Revendications

1. Composé de formule (I) :

$$R_1 - \underset{(O)_w}{\overset{}{S}} - (C_5H_6N_2R^+ X^- )_n H \qquad (I)$$

dans laquelle :

$R_1$ désigne un radical alkyle linéaire ou ramifié, ayant 12 à 18 atomes de carbone;
R désigne un radical méthyle, éthyle, hydroxyéthyle, benzyle;
$X^-$ désigne un anion minéral ou organique;
n est un nombre entier ou décimal compris entre 2 et 15;
w vaut 0, 1 ou 2;
le groupement $[C_5H_6N_2R^+]$ représentant les structures suivantes prises en mélange ou séparément :

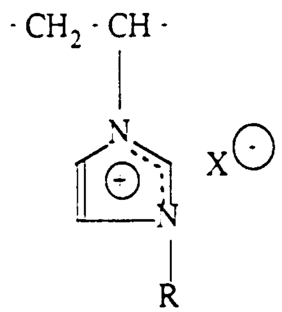
;
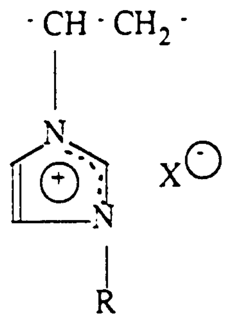

2. Composé selon la revendication 1, caractérisé par le fait que $X^-$ est choisi parmi $Cl^-$, $Br^-$, $I^-$, $CH_3\,OSO_3^-$, $C_2H_5OSO_3^-$, $CH_3\,SO_3^-$,

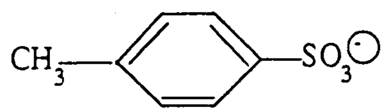

3. Composé selon la revendication 1 ou 2, caractérisé par le fait que le radical R désigne méthyle et $X^-$ représente l'anion $CH_3\,OSO_3^-$ ou R désigne benzyle et X désigne $Cl^-$.

4. Procédé de préparation des composés de formule (I) selon l'une quelconque des revendications 1 à 3, caractérisé par le fait qu'il consiste à effectuer, sous atmosphère inerte, en milieu solvant inerte, une réaction d'addition radicalaire en présence d'un initiateur de radicaux libres, d'un mercaptan de formule $R_1SH$, dans laquelle $R_1$ a la même signification indiquée dans la revendication 1, sur une ou plusieurs molécules de 1-vinylimidazole, pour obtenir un composé de formule (II) suivante :

$$R_1S(C_5H_6N_2)_n H \qquad (II)$$

où $C_5H_6N_2$ signifie les structures suivantes prises en mélange ou séparément :

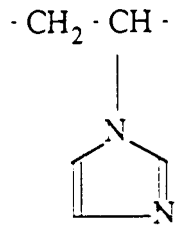
;
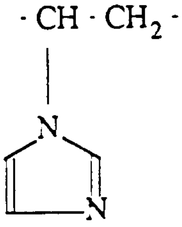
,

à quaterniser le composé ainsi obtenu en faisant réagir un alcoylant de formule RX, où R et X ont la même signification indiquée dans la revendication 1, en présence d'un solvant inerte, puis dans le cas où w vaut 1 ou 2, à oxyder le produit obtenu avec de l'eau oxygénée à une température comprise entre 20 et 50°C.

5. Composé répondant à la formule (II) :

$$R_1S(C_5H_6N_2)_n-H \qquad (II)$$

dans laquelle :

$R_1$ désigne un radical alkyle linéaire ou ramifié, ayant 12 à 18 atomes de carbone;
n est compris entre 2 et 15 ou représente une valeur statistique comprise entre 2 et 15;
$C_5H_6N_2$ signifiant les structures suivantes prises en mélange ou séparément :

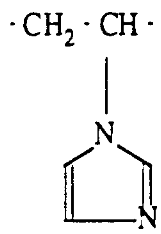
;
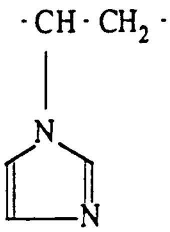

6. Utilisation des composés de formule (I) selon l'une quelconque des revendications 1 à 3, comme agents biocides ou agents conservateurs, dans des applications non-thérapeutiques.

7. Composition pour le soin ou le traitement des matières kératiniques humaines, en particulier des cheveux, de la peau, des ongles ou des muqueuses, caractérisée par le fait qu'elle contient dans un milieu physiologiquement acceptable, au moins un composé de formule (I) selon l'une quelconque des revendications 1 à 3.

8. Composition selon la revendication 7, caractérisée par le fait qu'elle contient le composé de formule (I) dans des concentrations comprises entre 0,1 et 10% en poids par rapport au poids total de la composition.

9. Composition selon la revendication 7 ou 8, caractérisée par le fait qu'elle se présente sous forme de solutions aqueuses, hydroalcooliques, ou alcooliques; d'émulsions se présentant sous forme de lait ou de crème; de mousse; de gel; de pâte; de stick; de spray; de dispersion vésiculaire ou conditionnée en aérosol.

10. Composition selon l'une quelconque des revendications 7 à 9, caractérisée par le fait qu'elle contient de l'eau, un mélange d'eau et de solvant(s) ou bien un solvant, le solvant étant choisi parmi les monoalcools en $C_1$-$C_4$ ou les polyalcools, et présent dans des proportions comprises entre 0 et 50%.

11. Composition selon l'une quelconque des revendications 7 à 10, caractérisée par le fait qu'elle contient en plus des huiles, des cires naturelles ou synthétiques, des alcools gras, des silicones, des tensio-actifs non ioniques, cationiques, faiblement anioniques, amphotères ou zwittérioniques, des polymères d'origine naturelle, ou synthétiques, des conditionneurs, des stabilisants de mousse, des épaississants, des nacrants, des stérols, des sels, des filtres solaires, des conservateurs autres que ceux de formule (I), notamment ceux de la famille des isothiazolones, des parfums, des colorants, des hydratants.

**12.** Composition pharmaceutique destinée à être appliquée par voie topique sur la peau pour le traitement des maladies cutanées d'origine bactérienne, mycobactérienne ou dues à des implantations de levures pathogènes, caractérisée par le fait qu'elle est constituée d'une composition telle que définie selon l'une quelconque des revendications 7 à 11.

**13.** Procédé de traitement cosmétique des cheveux et du cuir chevelu, caractérisé par le fait qu'on applique sur les cheveux ou le cuir chevelu, au moins une composition capillaire selon l'une quelconque des revendications 7 à 11, suivie éventuellement d'un rinçage à l'eau .

**14.** Utilisation des composés de formule (I) selon l'une quelconque des revendications 1 à 3 pour la préparation d'un médicament destiné au traitement des maladies d'origine bactérienne, mycobactérienne ou dues à des implantations de levures pathogènes.

**15.** Utilisation des composés de formule (I) selon l'une quelconque des revendications 1 à 3, comme agent tensio-actif.

**16.** Utilisation des composés de formule (I) selon l'une quelconque des revendications 1 à 3, comme agent de conditionnement des cheveux ou de la peau.

**Claims**

**1.** A compound of the formula (I):

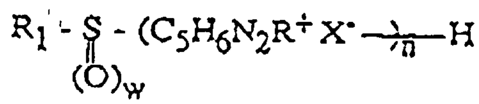

(I)

in which:

$R_1$ denotes a linear or branched alkyl radical having 12 to 18 carbon atoms;
R denotes a methyl, ethyl, hydroxyethyl or benzyl radical;
$X^-$ denotes an inorganic or organic anion;
n is an integer or decimal between 2 and 15;
w equals 0, 1 or 2;
the group $[C_5H_6N_2R^+]$ representing the following structures, taken mixed or separately:

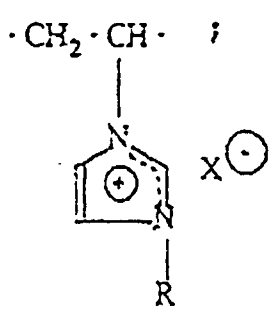

;

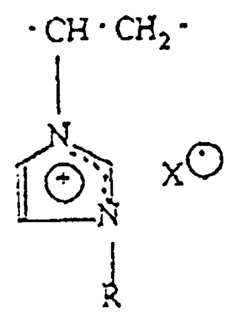

2. A compound as claimed in claim 1, wherein $X^-$ is chosen from $Cl^-$, $Br^-$, $I^-$, $CH_3OSO_3^-$, $C_2H_5OSO_3^-$, $CH_3SO_3^-$,

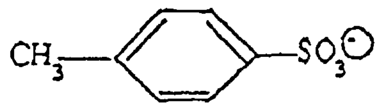

3. A compound as claimed in claim 1 or 2, wherein the radical R denotes methyl and $X^-$ represents the $CH_3OSO_3^-$ anion, or R denotes benzyl and X denotes $Cl^-$.

4. A process for preparing the compounds of formula (I) as claimed in any one of claims 1 to 3, which consists in performing, under an inert atmosphere, in an inert solvent medium and in the presence of a free-radical initiator, a free-radical addition reaction of a mercaptan of formula $R_1SH$, in which $R_1$ has the same meaning as stated in claim 1, with one or more molecules of 1-vinylimidazole, to obtain a compound of the following formula (II):

$$R_1S(C_5H_6N_2)_{\overline{n}}H \qquad (II)$$

where $C_5H_6N_2$ signifies the following structures, taken mixed or separately:

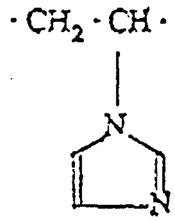 ; 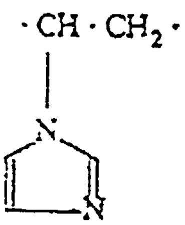

in quaternizing the compound thereby obtained by reacting an alkylating agent of formula RX where R and X have the meaning stated in claim 1, in the presence of an inert solvent, and then, in the case where w equals 1 or 2, in oxidizing the product obtained with hydrogen peroxide at a temperature of between 20° and 50°C.

5. A compound corresponding to the formula (II):

$$R_1S(C_5H_6N_2)_{\overline{n}}H \qquad (II)$$

in which:

$R_1$ denotes a linear or branched alkyl radical having 12 to 18 carbon atoms;
n is between 2 and 15 or represents a statistical value between 2 and 15;

$C_5H_6N_2$ signifying the following structures, taken mixed or separately:

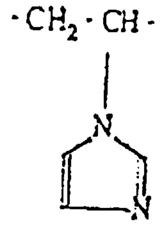
;
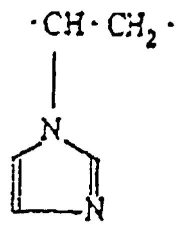

6. The use of the compounds of formula (I) as claimed in any one of claims 1 to 3, as biocidal agents or preservatives in non-therapeutic applications.

7. A composition for the care or treatment of human keratinous substances, especially the hair, skin, nails or mucosae, which contains at least one compound of formula (I) as claimed in any one of claims 1 to 3 in a physiologically acceptable medium.

8. The composition as claimed in claim 7, which contains the compound of formula (I) in concentrations of between 0.1 and 10% by weight relative to the total weight of the composition.

9. The composition as claimed in claim 7 or 8, which takes the form of aqueous, aqueous-alcoholic or alcoholic solutions; emulsions which take the form of a milk or cream; a foam; a gel; a paste; a stick; a spray; a vesicular dispersion; or is packaged as an aerosol.

10. The composition as claimed in any one of claims 7 to 9, which contains water, a mixture of water and solvent(s) or alternatively a solvent, the solvent being chosen from $C_1$-$C_4$ monohydric alcohols or polyhydric alcohols, and present in proportions of between 0 and 50%.

11. The composition as claimed in any one of claims 7 to 10, which contains, in addition, oils, natural or synthetic waxes, fatty alcohols, silicones, nonionic, cationic, weakly anionic, amphoteric or zwitterionic surfactants, polymers of natural origin or synthetic polymers, conditioners, foam stabilizers, thickeners, pearlescent agents, sterols, sun screens, preservatives other than those of formula (I), in particular those of the isothiazolone family, perfumes, colorings, hydrating agents.

12. A pharmaceutical composition intended for topical application to the skin for the treatment of skin diseases of bacterial or mycobacterial origin or those due to establishment of pathogenic yeasts, which consists of a composition as defined according to any one of claims 7 to 11.

13. A process for the cosmetic treatm ent of the hair and scalp, wherein at least one hair-care composition as claimed in any one of claims 7 to 11 is applied to the hair or scalp, optionally followed by a rinse with water.

14. The use of the compounds of formula (I) as claimed in any one of claims 1 to 3 for the preparation of a medicinal product intended for the treatment of diseases of bacterial or mycobacterial origin or those due to establishment of pathogenic yeasts.

15. The use of the compounds of formula (I) as claimed in any one of claims 1 to 3, as a surfactant.

16. The use of the compounds of formula (I) as claimed in any one of claims 1 to 3, as a conditioner for the hair or skin.

## Patentansprüche

1. Verbindung der Formel (I):

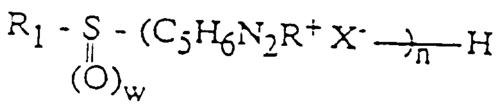

(I)

worin gilt:
$R_1$ bedeutet einen linearen oder verzweigten Alkylrest, der 12 bis 18 Kohlenstoffatome aufweist;
R bedeutet einen Methyl-, Ethyl-, Hydroxyethyl- oder Benzylrest;
$X^-$ bedeutet ein mineralisches oder organisches Anion;
n ist eine ganze Zahl oder Dezimalzahl von 2 bis 15;
w beträgt 0, 1 oder 2,
wobei die Gruppierung $[C_5H_6N_2R^+]$ die folgenden Strukturen, gemischt oder getrennt, darstellt:

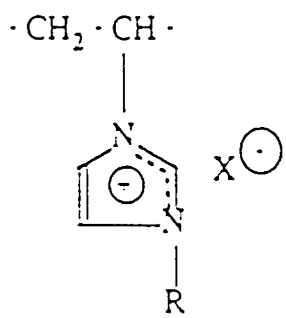

;

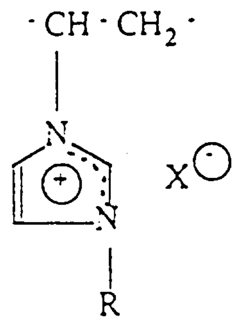

2. Verbindung gemäß Anspruch 1,
dadurch **gekennzeichnet**, daß
$X^-$ aus $Cl^-$, $Br^-$, $J^-$, $CH_3OSO_3^-$, $C_2H_5OSO_3^-$, $CH_3SO_3^-$ und aus

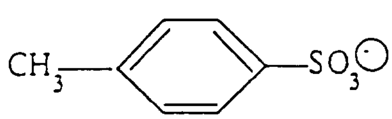

ausgewählt ist.

3. Verbindung gemäß Anspruch 1 oder 2,
dadurch **gekennzeichnet**, daß
der Rest R den Methylrest und $X^-$ das Anion $CH_3OSO_3^-$ oder R den Benzylrest und $X^-$ $Cl^-$ bedeuten.

4. Verfahren zur Herstellung von Verbindungen der Formel (I) gemäß jedem der Ansprüche 1 bis 3,
dadurch **gekennzeichnet**, daß
man in einer Inertatmosphäre in inertem Lösungsmittelmilieu in Gegenwart eines Initiators freier Radikale eine Additionsreaktion eines Mercaptans der Formel $R_1SH$, worin $R_1$ die in Anspruch 1 angegebene Bedeutung hat, mit

1-Vinylimidazol durchführt, um eine Verbindung der folgenden Formel (II) zu erhalten:

$$R_1S(C_5H_6N_2)_n\!-\!H \quad \text{(II)}$$

worin $C_5H_6N_2$ die folgenden Strukturen, gemischt oder getrennt, bedeutet:

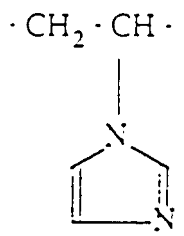 ; 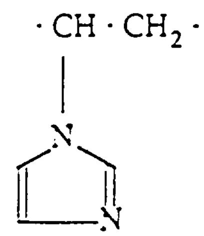

daß man die so erhaltene Verbindung mit einem Alkylierungsmittel der Formel RX, worin R und X die in Anspruch 1 angegebene Bedeutung haben, in Gegenwart eines inerten Lösungsmittels reagieren läßt und man im Fall, daß w 1 oder 2 beträgt, das erhaltene Produkt mit einem wässrigen Oxidationsmittel bei einer Temperatur von 20 bis 50°C oxidiert.

**5.** Verbindung gemäß der Formel (II):

$$R_1S(C_5H_6N_2)_n\!-\!H \quad \text{(II)}$$

worin gilt:
$R_1$ bedeutet einen linearen oder verzweigten Alkylrest mit 12 bis 18 Kohlenstoffatomen;
n beträgt 2 bis 15 oder stellt einen statistischen Wert von 2 bis 15 dar,
wobei $C_5H_6N_2$ die folgenden Strukturen, gemischt oder getrennt, bedeutet:

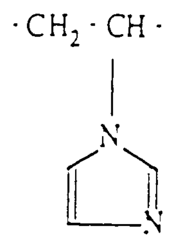 ; 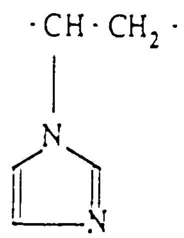

**6.** Verwendung von Verbindungen der Formel (I) gemäß jedem der Ansprüche 1 bis 3 als biozide Mittel oder Konservierungsmittel in nicht-therapeutischen Anwendungen.

**7.** Zusammensetzung zur Pflege oder Behandlung menschlicher keratinischer Materien, insbesondere der Haare, der Haut, der Nägel, oder von Schleimhäuten,
dadurch **gekennzeichnet**, daß
sie in einem physiologisch geeigneten Milieu mindestens eine Verbindung der Formel (I) gemäß jedem der Ansprüche 1 bis 3 enthält.

8. Zusammensetzung gemäß Anspruch 7,
dadurch **gekennzeichnet**, daß
sie die Verbindung der Formel (I) in Konzentrationen von 0,1 bis 10 Gew.% enthält, bezogen auf das Gesamtgewicht der Zusammensetzung.

9. Zusammensetzung gemäß Anspruch 7 oder 8,
dadurch **gekennzeichnet**, daß
sie in Form wässriger, hydroalkoholischer oder alkoholischer Lösungen, von Emulsionen in Form einer Milch oder Creme, in Form von Schaum, Gel oder Paste, eines Stabes, Spray, einer bläschenartigen Dispersion oder als Aerosol zubereitet vorliegt.

10. Zusammensetzung gemäß jedem der Ansprüche 7 bis 9,
dadurch **gekennzeichnet**, daß
sie Wasser, eine Mischung aus Wasser und Lösungsmittel(n) oder auch ein Lösungsmittel enthält, wobei das Lösungsmittel aus $C_{1-4}$-Monoalkoholen oder Polyalkoholen ausgewählt ist und in Mengenanteilen von 0 bis 50% vorliegt.

11. Zusammensetzung gemäß einem der Ansprüche 7 bis 10,
dadurch **gekennzeichnet**, daß
sie zusätzlich Öle, natürliche oder synthetische Wachse, Fettalkohole, Silikone, nicht-ionische, kationische, schwach anionische, amphotere oder zwitterionische oberflächenaktive Mittel, natürliche oder synthetische Polymere, Konditioniermittel, Schaumstabilisatoren, Verdickungsmittel, Perlmuttglanzmittel, Sterole, Salze, Sonnenfilterstoffe, Konservierungsstuffe, die sich von denen der Formel (I) unterscheiden, insbesondere diejenigen der Familie der Isothiazolone, Parfüm-Produkte, Färbemittel und Hydratisiermittel enthält.

12. Pharmazeutische Zusammensetzung zur topischen Aufbringung auf die Haut zur Behandlung von Hautkrankheiten bateriellen, mykrobakteriellen Ursprungs oder aufgrund von Implantationen pathogener Hefen,
dadurch **gekennzeichnet**, daß
sie durch eine gemäß jedem der Ansprüche 7 bis 11 definierte Zusammensetzung dargestellt ist.

13. Verfahren zur kosmetischen Behandlung der Haare oder behaarter Haut,
dadurch **gekennzeichnet**, daß
man auf die Haare oder die behaarte Haut mindestens eine Haarmittelzusammensetzung gemäß einem der Ansprüche 7 bis 11 aufbringt, worauf gegebenenfalls eine Spülung mit Wasser erfolgt.

14. Verwendung der Verbindungen der Formel (I) gemäß jedem der Ansprüche 1 bis 3 zur Herstellung eines Medikaments zur Behandlung von Krankheiten bakteriellen, mykobakteriellen Ursprungs oder aufgrund von Implantationen pathogener Hefen.

15. Verwendung der Verbindungen der Formel (I) gemäß jedem der Ansprüche 1 bis 3 als oberflächenaktives Mittel.

16. Verwendung der Verbindungen der Formel (I) gemäß jedem der Ansprüche 1 bis 3 als Konditioniermittel der Haare oder der Haut.